(19) **Europäisches Patentamt / European Patent Office / Office européen des brevets**

(11) **EP 4 775 636 A1**

(12) **EUROPEAN PATENT APPLICATION**
published in accordance with Art. 153(4) EPC

(43) Date of publication:
**15.07.2026 Bulletin 2026/29**

(21) Application number: **24862590.7**

(22) Date of filing: **22.08.2024**

(51) International Patent Classification (IPC):
**C08L 101/14** (2006.01)   **A61F 13/15** (2006.01)
**A61F 13/53** (2006.01)   **B01J 20/26** (2006.01)
**C08J 3/20** (2006.01)   **C08K 3/34** (2006.01)
**C08K 5/00** (2006.01)   **C08K 5/49** (2006.01)
**C08K 5/092** (2006.01)

(52) Cooperative Patent Classification (CPC):
**A61F 13/15; A61F 13/53; B01J 20/26; C08J 3/20;
C08K 3/34; C08K 5/00; C08K 5/092; C08K 5/49;
C08L 101/14**

(86) International application number:
**PCT/JP2024/029757**

(87) International publication number:
**WO 2025/052937 (13.03.2025 Gazette 2025/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **05.09.2023 JP 2023143714**

(71) Applicant: **Nippon Shokubai Co., Ltd.
Osaka-shi, Osaka 541-0043 (JP)**

(72) Inventors:
• **MATSUI, Kentaro**
  **Himeji-shi, Hyogo 671-1282 (JP)**
• **FUJIMOTO, Taku**
  **Himeji-shi, Hyogo 671-1282 (JP)**

(74) Representative: **Henkel & Partner mbB
Patentanwaltskanzlei, Rechtsanwaltskanzlei
Maximiliansplatz 21
80333 München (DE)**

(54) **WATER-ABSORBING RESIN COMPOSITION**

(57) [Problem] To provide a novel water-absorbent resin composition that can further improve the reduction of urine odor as compared with conventional water-absorbent resin compositions and can also suppress the recurrence of bad odors in a high temperature environment.

[Solution] A water-absorbent resin composition includes a water-absorbent resin, a copper silicate, and a chelating agent.

**EP 4 775 636 A1**

**Description**

Technical Field

The present invention relates to a water-absorbent resin composition.

Background Art

Water-absorbent resins are widely used as a material in sanitary products such as disposable diapers, sanitary napkins, and incontinence pads for the purpose of absorbing body fluids such as urine and blood, and are a main constituent material of these sanitary products. In recent years, the demand for disposable diapers for adults has been increasing due to the aging of society, and in association therewith, the demand for imparting deodorizing properties to water-absorbent resins has been growing.

Thus, in recent years, compositions that contain a water-absorbent resin and an inorganic antibacterial agent in which an antibacterial metal such as silver, copper, or zinc is supported on an inorganic compound have been proposed as compositions that suppress the generation of odors (for example, see Patent Document 1). The inorganic antibacterial agent used in these compositions exhibits long-lasting antibacterial activity, and thus is deemed suitable for use in sanitary materials and the like.

Citation List

Patent Document

Patent Document 1: WO 2006/046496 A1

Summary of Invention

Technical Problem

However, in environments where the storage temperature is high, an issue of once-removed bad odors returning occurs. For example, when a used diaper is thrown into a trash can and stored in a high temperature environment such as during the summer, the issue of the bad odor returning occurs. For example, when a used diaper is thrown into a trash can and stored in a high temperature environment such as during the summer, the issue of the bad odor returning occurs.

Therefore, an issue to be addressed is to provide a novel water-absorbent resin composition that can further improve the reduction of urine odor as compared with conventional water-absorbent resin compositions, and can also suppress the recurrence of bad odors in a high temperature environment.

Solution to Problem

One aspect for achieving the above-described object is a water-absorbent resin composition containing a water-absorbent resin, a copper silicate, and a chelating agent.

Advantageous Effects of Invention

According to the present invention, a novel water-absorbent resin composition that can further improve the reduction of urine odor as compared with conventional water-absorbent resin compositions and can also suppress the recurrence of bad odors in a high temperature environment can be provided.

Description of Embodiments

Hereinafter, the present invention will be described with reference to the best embodiment. Throughout the present specification, it is to be understood that the expression of a singular form includes also a concept of a plural form thereof, unless otherwise specified. Thus, it should be understood that a singular article (for example, "a", "an", "the", and the like in the case of English) includes a concept of its plural form unless otherwise specified. Unless otherwise specified, operations and measurements of physical properties and the like are carried out at room temperature (20 to 25°C). Moreover, "weight" and "mass", "parts by weight" and "parts by mass", "%", "mass%" and "wt.%", and "ppm by mass" and "ppm by wt." are treated as being synonymous, respectively. It should also be understood that the terms used herein are used having the meanings commonly used in the art, unless otherwise specified. Accordingly, unless defined otherwise, all technical and scientific terms used herein have the same meanings as commonly understood by those skilled in the art to which the present invention belongs. If there is any conflict, the present specification (including definitions) will prevail. The present invention is not limited to the following embodiments, and can be variously modified within the scope of the claims. It must also be understood that all and any combinations of lower and upper limit values disclosed herein are also disclosed. That is, it should be understood that all and any combinations thereof can serve as a basis for amendments. It should also be understood that all and any combinations of the embodiments presented in the present application are also disclosed herein. That is, it should be understood that all and any combinations thereof can serve as a basis for amendments.

One aspect for achieving the above-described object is a water-absorbent resin composition containing a water-absorbent resin, a copper silicate, and a chelating agent. Through such a configuration, a novel water-absorbent resin composition that can further improve the reduction of urine odor as compared with conventional water-absorbent resin compositions and can also suppress the recurrence of bad odors in a high temperature environment (for example, 40°C) can be provided.

[1] Definition of Terms

[1-1] Water-Absorbent Resin, Base Polymer, and Water-Absorbent Resin Composition

The term "water-absorbent resin" in the present invention refers to a polymer gelling agent that is water-swellable and water-insoluble, and the water-absorbent resin is generally in a powder form. In addition, the term "water-swellable" means that the absorption capacity without pressure (also referred to as centrifuge retention capacity (CRC)) as defined by the EDANA WSP 241.3(10) test method is 5 g/g or greater, and the term "water-insoluble" means that the soluble content (Ext) defined by the EDANA WSP 270.3(10) test method is 50 mass% or less.

The "water-absorbent resin" is preferably a hydrophilic crosslinked polymer (so-called internally crosslinked polymer) obtained by crosslinking and polymerizing an unsaturated monomer having a carboxyl group, but the entire amount (100 mass%) thereof is not necessarily a crosslinked polymer.

Also, generally, the water-absorbent resin may refer to a "polymer in which only the inside is crosslinked (that is, a polymer in which the crosslinking density of the inside and the crosslinking density of the surface are substantially the same)" or a "polymer in which the inside and the surface are crosslinked (that is, a polymer in which the crosslinking density of the surface is relatively high with respect to the crosslinking density of the inside)". A polymer in which only the inside is crosslinked may be denoted as a "base polymer".

The "water-absorbent resin composition" of the present invention means a composition containing a water-absorbent resin, a copper silicate, and a chelating agent and optionally containing other components, but put simply, the "water-absorbent resin composition" means a resin that is water absorbent and is in a state of being shippable as a final product. Therefore, when the copper silicate, the chelating agent, and, as necessary, other additives are contained in a water-absorbent resin, a "water-absorbent resin composition" is obtained. In the present specification, the water-absorbent resin composition may be simply referred to as a "water-absorbing agent".

[1-2] "EDANA", "WSP", and "NWSP"

"EDANA" is an abbreviation for the European Disposables and Nonwovens Association. The term "WSP" is an abbreviation for Worldwide Strategic Partners and indicates a world standard measurement method provided by EDANA for water-absorbent resins. "NWSP" is an abbreviation for "Non-Woven Standard Procedures - Edition 2015". In the present invention, unless otherwise specified, the physical properties of the water-absorbent resin are measured in accordance with the WSP original procedures (revised in 2010/known literature).

[1-2-1] "CRC" (WSP 241. 3(10))

"CRC", which means the absorption capacity without pressure, is an abbreviation for Centrifuge Retention Capacity and means an absorption capacity under a condition of no pressure. Specifically, CRC refers to the absorption capacity (unit: g/g) measured after 0.2 g of a substance to be measured (for example, a water-absorbing agent or a water-absorbent resin) is placed in a non-woven fabric bag, after which the bag is immersed in a large excess of a 0.9 mass% aqueous sodium chloride solution for 30 minutes to allow the bag to freely swell, and then the bag is dewatered for 3 minutes using a centrifuge (250 G).

[1-2-2] "AAP" (NWSP 242.0.R2(15))

"AAP", which means the absorption capacity under pressure, is an abbreviation for Absorption Against Pressure and means the capacity for water absorption of a substance to be measured (for example, a water-absorbing agent or a water-absorbent resin) under a condition of pressurization (the water absorption capacity may also be referred to as the "absorption capacity"). Specifically, AAP 0.3 psi (2.06 kPa) refers to the water absorption capacity (unit: g/g) measured after 0.9 g of a substance to be measured (for example, a water-absorbing agent or a water-absorbent resin) is placed in a large excess of a 0.9 mass% aqueous sodium chloride solution for 1 hour to allow the substance to swell under a load of 2.06 kPa (= 21 g/cm$^2$ = 0.3 psi).

[2] Water-Absorbent Resin Composition and Method for Producing Same

A water-absorbent resin composition according to one aspect of the present invention is a water-absorbent resin composition containing a water-absorbent resin, a copper silicate, and a chelating agent.

Water-Absorbent Resin

In one embodiment of the present invention, examples of the water-absorbent resin include polyacrylic acid (salt)-based water-absorbent resins, polysulfonic acid (salt)-based water-absorbent resins, maleic anhydride (salt)-based water-absorbent resins, polyacrylamide-based water-absorbent resins, polyvinyl alcohol-based water-absorbent resins, polyethylene oxide-based water-absorbent resins, polyaspartic acid (salt)-based water-absorbent resins, polyglutamic acid (salt)-based water-absorbent resins, polyalginic acid (salt)-based water-absorbent resins, starch-based water-absorbent resins, and cellulose-based water-absorbent resins. Among these, a polyacrylic acid (salt)-based water-absorbent resin is preferably used.

The term "polyacrylic acid (salt)-based water-absorbent resin" in the present embodiments of the present invention means a water-absorbent resin that contains, as a main component, an acrylic acid and/or a salt thereof (hereinafter, denoted as an "acrylic acid (salt)"). That is, the polyacrylic acid (salt)-based water-absorbent resin is a water-absorbent resin having a structural unit derived from an acrylic acid (salt) in the polymer and having a graft component as an optional component.

More specifically, the polyacrylic acid (salt)-based water-absorbent resin is a water-absorbent resin containing an acrylic acid (salt) at an amount of preferably from 50 mol% to 100 mol%, more preferably from 70 mol% to 100 mol%, still more preferably from 90 mol% to 100 mol%, and particularly preferably substantially 100 mol% with respect to all monomers (excluding, or course, an internal crosslinking agent) participating in the polymerization reaction.

Copper Silicate

The copper silicate of the present invention is a structure in which a copper ion in a copper salt and a silicate anion are chemically bonded. The copper silicate is not a mixture obtained by merely mixing a copper salt and silicic acid in a solid state, and is not a substance that supports copper using, as a base material, a silicic acid that does not contain copper. In one embodiment of the present invention, the copper silicate contains an oxygen atom. In one embodiment of the present invention, the copper silicate contains a sulfur atom. According to such embodiments, the effect of suppressing bad odors (particularly, a urine odor) and the effect of suppressing the recurrence of a bad odor in a high temperature environment become more remarkable. In one embodiment of the present invention, the copper silicate contains a sodium atom. In one embodiment of the present invention, the copper silicate contains an aluminum atom.

In one embodiment of the present invention, the copper silicate can be obtained, for example, by mixing, in water, a precursor compound (for example, a silicate) serving as a silicon source and a precursor compound (for example, an inorganic salt of copper or an organic salt of copper) serving as a copper source, and preferably mixing an adjusting agent (for example, sulfuric acid) that can adjust the liquid to an acidic state. The mixing amount may be adjusted so as to obtain, for example, the atomic ratio and mass ratio described below. The temperature when mixing may be approximately room temperature (for example, from 10 to 40°C), or heating may be carried out to an upper limit temperature of approximately 60°C as a guide. The mixing time may be, for example, from about 1 minute to about 3 days. Solids are filtered from the mixed solution, washed with ion-exchanged water, and dried, after which the dried solid is pulverized to obtain a powdery copper silicate.

In one embodiment of the present invention, the copper silicate comprises silicon atoms, copper atoms, sulfur atoms, oxygen atoms, and sodium atoms.

In one embodiment of the present invention, the atomic ratio of copper atoms in the copper silicate per 100 silicon atoms is 1 or greater, 2 or greater, 3 or greater, 4 or greater, or 5 or greater. In one embodiment of the present invention, the atomic ratio of copper atoms in the copper silicate per 100 silicon atoms is 10 or less, 9 or less, 8 or less, 7 or less, or 6 or less.

In one embodiment of the present invention, the atomic ratio of sulfur atoms in the copper silicate per 100 silicon atoms is 1 or greater, 2 or greater, 3 or greater, 4 or greater, or 5 or greater. In one embodiment of the present invention, the atomic ratio of sulfur atoms in the copper silicate per 100 silicon atoms is 10 or less, 9 or less, 8 or less, or 7 or less.

In one embodiment of the present invention, the atomic ratio of oxygen atoms in the copper silicate per 100 silicon atoms is 200 or greater, 250 or greater, 300 or greater, 350 or greater, 400 or greater, 450 or greater, or 500 or greater. In one embodiment of the present invention, the atomic ratio of oxygen atoms in the copper silicate per 100 silicon atoms is 900 or less, 850 or less, 800 or less, 750 or less, 700 or less, 650 or less, or 600 or less.

In one embodiment of the present invention, the atomic ratio of sodium atoms in the copper silicate per 100 silicon atoms is 5 or greater, 10 or greater, 15 or greater, or 20 or greater. In one embodiment of the present invention, the atomic ratio of sodium atoms in the copper silicate per 100 silicon atoms is 50 or less, 45 or less, 40 or less, 35 or less, 30 or less, or 25 or less.

In one embodiment of the present invention, the weight ratio of copper atoms in the copper silicate per 100 silicon atoms is 5 or greater, 7 or greater, 9 or greater, or 10 or greater. In an embodiment of the present invention, the weight ratio of copper atoms in the copper silicate per 100 silicon atoms is 20 or less, 18 or less, 16 or less, 14 or less, or 13 or less.

In one embodiment of the present invention, the weight ratio of sulfur atoms in the copper silicate per 100 silicon atoms is 1 or greater, 2 or greater, 3 or greater, 4 or greater, or 5 or greater. In one embodiment of the present invention, the weight ratio of sulfur atoms in the copper silicate per 100 silicon atoms is 10 or less, 9 or less, 8 or less, or 7 or less.

In one embodiment of the present invention, the weight ratio of oxygen atoms in the copper silicate per 100 silicon atoms is 100 or greater, 200 or greater, 250 or greater, or 300 or greater. In one embodiment of the present invention, the weight ratio of oxygen atoms in the copper silicate per 100 silicon atoms is 600 or less, 550 or less, 500 or less, 450 or less, 400 or less, or 350 or less.

In one embodiment of the present invention, the weight ratio of sodium atoms in the copper silicate per 100 silicon atoms is 5 or greater, 10 or greater, 15 or greater, or 17 or greater. In one embodiment of the present invention, the weight ratio of sodium atoms in the copper silicate per 100 silicon atoms is 50 or less, 45 or less, 40 or less, 35 or less, 30 or less, 25 or less, or 20 or less.

In one embodiment of the present invention, the copper silicate comprises silicon atoms, copper atoms, sulfur atoms, oxygen atoms, and aluminum atoms.

In one embodiment of the present invention, the atomic ratio of copper atoms in the copper silicate per 100 silicon atoms is 10 or greater, 15 or greater, 20 or greater, 25 or greater, 30 or greater, or 35 or greater. In one embodiment of the present invention, the atomic ratio of copper atoms in the copper silicate per 100 silicon atoms is 60 or less, 55 or less, 50 or less, 45 or less, or 40 or less.

In one embodiment of the present invention, the atomic ratio of sulfur atoms in the copper silicate per 100 silicon atoms is 0.01 or greater, 0.05 or greater, or 0.1 or greater. In one embodiment of the present invention, the atomic ratio of sulfur atoms in the copper silicate per 100 silicon atoms is 2 or less, 1 or less, 0.8 or less, or 0.5 or less.

In one embodiment of the present invention, the atomic ratio of oxygen atoms in the copper silicate per 100 silicon atoms is 400 or greater, 450 or greater, 500 or greater, 550 or greater, 600 or greater, or 650 or greater. In one embodiment of the present invention, the atomic ratio of oxygen atoms in the copper silicate per 100 silicon atoms is 1100 or less, 1000 or less, 900 or less, 850 or less, 800 or less, or 750 or less.

In one embodiment of the present invention, the atomic ratio of aluminum atoms in the copper silicate per 100 silicon atoms is 0.01 or greater, 0.05 or greater, 0.1 or greater, 0.5 or greater, 1 or greater, or 2 or greater. In one embodiment of the present invention, the atomic ratio of aluminum atoms in the copper silicate per 100 silicon atoms is 10 or less, 7 or less, 5 or less, 3 or less, 2.5 or less, or 2 or less.

In one embodiment of the present invention, the weight ratio of copper atoms in the copper silicate per 100 silicon atoms is 40 or greater, 50 or greater, 60 or greater, or 70 or greater. In one embodiment of the present invention, the weight ratio of copper atoms in the copper silicate per 100 silicon atoms is 200 or less, 150 or less, or 100 or less.

In one embodiment of the present invention, the weight ratio of sulfur atoms in the copper silicate per 100 silicon atoms is 0.001 or greater, 0.005 or greater, 0.008 or greater, or 0.01 or greater. In one embodiment of the present invention, the weight ratio of sulfur atoms in the copper silicate per 100 silicon atoms is 5 or less, 3 or less, 1 or less, or 0.5 or less.

In one embodiment of the present invention, the weight ratio of oxygen atoms in the copper silicate per 100 silicon atoms is 150 or greater, 200 or greater, 250 or greater, 300 or greater, 350 or greater, or 400 or greater. In one embodiment of the present invention, the weight ratio of oxygen atoms in the copper silicate per 100 silicon atoms is 700 or less, 600 or less, 500 or less, or 450 or less.

In one embodiment of the present invention, the weight ratio of aluminum atoms in the copper silicate per 100 silicon atoms is 0.01 or greater, 0.05 or greater, 0.1 or greater, 0.5 or greater, 1 or greater, or 2 or greater. In one embodiment of the present invention, the weight ratio of aluminum atoms in the copper silicate per 100 silicon atoms is 10 or less, 7 or less, 5 or less, 3 or less, 2.5 or less, or 2 or less.

In the present specification, the atomic ratio and the weight ratio can be calculated by carrying out SEM-EDS analysis using a scanning electron microscope.

In one embodiment of the present invention, copper accounts for 50 mass% or more, 60 mass% or more, or 70 mass% or more (with the upper limit being 100 mass%) of all the polyvalent metal components contained in the copper silicate.

In one embodiment of the present invention, the copper silicate consists of an amorphous structure. According to such an embodiment, the effect of suppressing a bad odor (particularly, a urine odor) can be improved. Whether the structure is amorphous can be confirmed, for example, by an X-ray crystal structure analysis method.

In one embodiment of the present invention, the volume-average particle size of the copper silicate is from 0.1 to 100 $\mu$m, from 0.5 to 50 $\mu$m, or from 1 to 20 $\mu$m. The volume-average particle size of the copper silicate can be measured with a laser diffraction-type particle size distribution measuring device.

In one embodiment of the present invention, the solubility of the copper silicate in ion-exchanged water at 25°C is 0.5 g/100 mL-$H_2O$ or less, 0.3 g/100 mL-$H_2O$ or less, or 0.1 g/100 mL-$H_2O$ or less. In such an embodiment, permeation of the copper silicate into water-absorbent resins can be reduced, and the desired effects of the present invention can thus be efficiently exhibited. As to solubility, for example, when 0.1 g of a substance to be tested is put into a 100 mL beaker, and 20 g of 20°C ion-exchanged water is added thereto, and if insoluble matter is visually confirmed 24 hours later, the solubility can be confirmed to be 0.5 g/100 mL-$H_2O$ or less. Similarly, whether the solubility is equal to or less than a certain level or equal to or greater than the certain level can be easily determined by changing the amount of water in the above-described method and visually checking whether insoluble matter is present.

In one embodiment of the present invention, when the copper silicate contains zinc, the mass ratio of the content of zinc to the content of silicon ((content of zinc)/(content of silicon)) in the copper silicate is less than 50/50, less than 10/50, or less than 1/50.

In one embodiment of the present invention, copper silicate does not contain zinc or zinc atoms.

In one embodiment of the present invention, the content of copper silicate relative to the water-absorbent resin is from 0.01 to 10 mass%, from 0.05 to 9 mass%, from 0.1 to 8 mass%, from 0.2 to 7 mass%, from 0.3 to 6 mass%, from 0.4 to 5 mass%, from 0.5 to 4 mass%, from 0.6 to 3 mass%, or from 0.7 to 2 mass%.

Chelating Agent

In one embodiment of the present invention, a chelating agent may be a compound having a chelating effect of capturing a metal. In one embodiment of the present invention, the chelating agent contains at least one compound selected from the group consisting of an amino polycarboxylic acid, an amino polyphosphoric acid, and salts thereof.

The term "poly-" means that a plurality of functional groups are present in one molecule, and the number of functional groups is preferably from 2 to 30, more preferably from 3 to 20, still more preferably from 4 to 10, and yet even more preferably from 5 to 9.

From the viewpoint of the influence on polymerization and the physical properties of the resulting water-absorbent resin composition, the molecular weight of the chelating agent is preferably from 100 to 5000, and more preferably from 150 to 1000.

Specific examples of the amino polycarboxylic acid or salts thereof (amino polycarboxylic acid-based chelating agent) include iminodiacetic acid, hydroxyethyl iminodiacetic acid, nitrilotriacetic acid, nitrilotripropionic acid, ethylenediamine tetraacetic acid, diethylenetriamine pentaacetic acid, triethylenetetraamine hexaacetic acid, trans-1,2-diaminocyclohexane tetraacetic acid, N,N-bis(2-hydroxyethyl)glycine, diaminopropanol tetraacetic acid, ethylenediamine dipropionic acid, N-hydroxyethylethylene diamine triacetic acid, glycol etherdiamine tetraacetic acid, diaminopropane tetraacetic acid, N,N'-bis(2-hydroxybenzyl)ethylenediamine-N,N'-diacetic acid, 1,6-hexamethylenediamine-N,N,N',N'-tetraacetic acid, and salts thereof.

Specific examples of the amino polyphosphoric acid or salts thereof (amino polyphosphoric acid-based chelating agent) include ethylenediamine-N,N'-di(methylene phosphinic acid), ethylenediamine tetra(methylene phosphinic acid), cyclohexanediamine tetra(methylenephosphonic acid), ethylenediamine-N,N'-diacetic acid-N,N'-di(methylenephosphonic acid), ethylenediamine-N,N'-di(methylenephosphonic acid), ethylenediamine tetra(methylenephosphonic acid), polymethylenediamine tetra(methylenephosphonic acid), diethylenetriamine penta(methylenephosphonic acid), and salts thereof.

Among these, from the viewpoint of suppressing bad odors, an amino polyvalent carboxylic acid-based chelating agent having four or more (or five or more) carboxyl groups or an amino polyvalent phosphoric acid-based chelating agent having four or more (or five or more) phosphonic acid groups is preferable. In one embodiment of the present invention, from the viewpoint of suppressing bad odors, diethylenetriamine pentaacetic acid, nitrilotriacetic acid, triethylenetetraamine hexaacetic acid, ethylenediamine tetra(methylenephosphonic acid), and salts thereof are more preferable.

In one embodiment of the present invention, examples of salts of the chelating agents include, but are not limited to, sodium and the like.

In one embodiment of the present invention, the chelating agent may be a compound exemplified in US 6599989 B2, US 6469080 B2, EP 2163302 A1, and the like.

In one embodiment of the present invention, the content of the chelating agent relative to the water-absorbent resin is from 10 to 1000 ppm by mass, from 20 to 800 ppm by mass, from 30 to 600 ppm by mass, from 40 to 500 ppm by mass, from 50 to 300 ppm by mass, from 60 to 200 ppm by mass, or from 70 to 150 ppm by mass.

Hereinafter, a preferred method for producing the water-absorbent resin composition will be described. However, the method for producing the water-absorbent resin composition according to one aspect of the present invention is of course not limited to the following production method.

[2-1] Step for Preparing Aqueous Monomer Solution

The present step is a step for preparing an aqueous monomer solution containing a monomer and at least one type of internal crosslinking agent, the monomer containing an acrylic acid (salt) as a main component. The term "main component" means that the usage amount (content) of the acrylic acid (salt) based on all monomers (excluding the internal crosslinking agent) to be subjected to polymerization reaction is typically 50 mol% or greater, preferably 70 mol% or greater, and more preferably 90 mol% or greater (the upper limit is 100 mol%). Note that a slurry liquid of the monomer may be used within a scope that does not impact the water absorption performance of the water-absorbing agent to be obtained as a final product. However, in the present specification, an aqueous monomer solution will be described for simplicity.

Acrylic Acid (Salt)

In the embodiments of the present invention, from the viewpoint of the physical properties of the water-absorbing agent and productivity, a known acrylic acid (salt) is preferably used as a monomer (also referred to as a polymerizable monomer). The known acrylic acid may contain a trace amount of a component such as a polymerization inhibitor and/or an impurity. For the polymerization inhibitor, preferably methoxyphenols, and more preferably p-methoxyphenols are used. From the viewpoint of the polymerizability of the acrylic acid and the color tone of the water-absorbing agent, the content (concentration) of the polymerization inhibitor in the acrylic acid is preferably 200 ppm (by mass) or less, more preferably from 10 ppm (by mass) to 160 ppm (by mass), and even more preferably from 20 ppm (by mass) to 100 ppm (by mass). For the impurities, in addition to organic compounds such as acetic acid, propionic acid, and furfural, each of the compounds described in US 2008/0161512 A1 may also be contained in the acrylic acid that is in the embodiments of the present invention.

Moreover, examples of acrylates include salts (for example, sodium acrylate) obtained by neutralizing the above-described acrylic acid with a basic compound described below. The acrylates may be commercially available acrylates (for example, sodium acrylate), or may be one obtained by neutralizing acrylic acid.

Basic Compound

A basic compound according to the embodiments of the present invention refers to a compound that exhibits a basic property, and specific examples thereof include basic compounds such as sodium hydroxide. Commercially available sodium hydroxide contains a heavy metal such as zinc, lead, or iron in an order of ppm (by mass), and thus strictly speaking, such sodium hydroxide can be expressed as a composition. However, in the present invention, such a composition is also included in the category of a basic compound.

The specific examples of the basic compound include a carbonate or a hydrogen carbonate of an alkali metal, a hydroxide of an alkali metal, ammonia, and an organic amine. Among these, a strongly basic compound is selected from the viewpoint of the water absorption performance of the water-absorbing agent. Therefore, hydroxides of alkali metals such as sodium, potassium, and lithium are preferable, and sodium hydroxide is more preferable. Note that the basic compound is preferably an aqueous solution from the viewpoint of ease of handling.

Neutralization

When using, as the acrylate, a salt obtained by neutralizing acrylic acid, the timing at which the neutralization is carried out is not particularly limited and may be before polymerization, during polymerization, or after polymerization, and the neutralization may be carried out at multiple timings or at multiple locations. Moreover, from the viewpoint of production efficiency of the water-absorbing agent, neutralization is preferably carried out in a continuous manner.

In a case in which an acrylic acid (salt) is used in the present invention, the rate of neutralization relative to an acid group of the monomer is preferably from 10 mol% to 90 mol%, more preferably from 40 mol% to 85 mol%, still more preferably from 50 mol% to 80 mol%, and particularly preferably from 60 mol% to 78 mol%. By setting the rate of neutralization within this range, a decrease in the water absorption performance of the water-absorbing agent can be suppressed.

The above-described ranges for the rate of neutralization may be applied to any of the above-described neutralization timings, namely before polymerization, during polymerization, and after polymerization. The same applies to the water-absorbing agent as a final product.

Other Monomers

In the present invention, a monomer (hereinafter, referred to as "other monomer") other than the above-described acrylic acid (salt) can be used in combination with the acrylic acid (salt) as necessary.

Specific examples of the other monomer include anionic unsaturated monomers and salts thereof, such as maleic acid (maleic anhydride), itaconic acid, cinnamic acid, vinyl sulfonic acid, allyl toluene sulfonic acid, vinyl toluene sulfonic acid, styrene sulfonic acid, 2-(meth)acrylamide-2-methylpropane sulfonic acid, 2-(meth)acryloyl ethane sulfonic acid, 2-(meth)acryloyl propane sulfonic acid, and 2-hydroxyethyl (meth)acryloyl phosphate; mercaptan group-containing unsaturated monomers; phenolic hydroxyl group-containing unsaturated monomers; amide group-containing unsaturated monomers such as (meth)acrylamide, N-ethyl(meth)acrylamide, and N,N-dimethyl(meth)acrylamide; and amino group-containing unsaturated monomers such as N,N-dimethylaminoethyl (meth)acrylate, N,N-dimethylaminopropyl (meth)acrylate, and N,N-dimethylaminopropyl (meth)acrylamide. The other monomers also include water-soluble or hydrophobic unsaturated monomers. When the other monomer is used, the amount thereof based on all monomers (excluding the internal crosslinking agent) is preferably 30 mol% or less, more preferably 10 mol% or less, and even more preferably 5 mol% or less (lower limit: 0 mol%).

Internal Crosslinking Agent

In a preferred production method, an internal crosslinking agent is used. Specific examples of the internal crosslinking agent include N,N'-methylenebis(meth)acrylamides, (poly)ethylene glycol di(meth)acrylates, (poly)propylene glycol di(meth)acrylates, trimethylolpropane di(meth)acrylates, trimethylolpropane tri(meth)acrylates, glycerin tri(meth)acrylates, glycerin acrylate methacrylates, ethylene oxide-modified trimethylolpropane tri(meth)acrylates, pentaerythritol tetra(meth)acrylates, dipentaerythritol hexa(meth)acrylates, triallyl cyanurate, triallyl isocyanurate, triallyl phosphate, triallylamine, poly(meth)allyloxyalkanes, (poly)ethylene glycol diglycidyl ethers, glycerol diglycidyl ether, ethylene glycol, polyethylene glycols, propylene glycols, glycerin, pentaerythritol, ethylenediamine, polyethyleneimines, and glycidyl (meth)acrylates. Among these internal crosslinking agents, at least one type of internal crosslinking agent is selected in consideration of reactivity and the like. From viewpoints such as the water absorption performance of the water-absorbing agent, an internal crosslinking agent having two or more polymerizable unsaturated groups is preferably selected, an internal crosslinking agent that is thermally decomposable at the drying temperature is more preferably selected, and an internal crosslinking agent having two or more polymerizable unsaturated groups having a (poly)alkylene glycol structure is even more preferably selected.

Specific examples of the polymerizable unsaturated group include an allyl group and a (meth)acrylate group, and a (meth)acrylate group is more preferable. Moreover, a specific example of the (poly)alkylene glycol structure is polyethylene glycol. The number of alkylene glycol units (hereinafter, the number thereof may be denoted as n) is preferably from 1 to 100, more preferably from 6 to 50, still more preferably from 6 to 20, and most preferably from 6 to 10.

The amount of the internal crosslinking agent based on all the monomers (excluding of course the internal crosslinking agent) is preferably from 0.0001 mol% to 10 mol%, more preferably from 0.001 mol% to 5 mol%, and even more preferably from 0.01 mol% to 1 mol%. By setting the amount within this range, a water-absorbent resin and a water-absorbing agent having the desired water absorption performance are obtained. Preferably, adjustment of the amount of the internal crosslinking agent should be considered in order to set the gel bulk density of the water-absorbent resin and the water-absorbing agent to within a prescribed range and suppress an increase in the water-soluble content and a decrease in the absorption capacity due to a decrease in the gel strength.

The internal crosslinking agent is preferably added in advance when preparing the aqueous monomer solution, and in this case, the crosslinking reaction is carried out simultaneously with the polymerization reaction. On the other hand, the polymerization reaction may be initiated without adding the internal crosslinking agent, and the internal crosslinking agent may then be added during or after the polymerization reaction to cause a crosslinking reaction. These techniques may also be used in combination.

Substances to Be Added to Aqueous Monomer Solution

In the embodiments of the present invention, any of the following substances can be added to the aqueous monomer solution at one or more timings selected from: when the aqueous monomer solution is prepared; during the polymerization reaction and the crosslinking reaction; or after the polymerization reaction and the crosslinking reaction, from the viewpoint of improving the physical properties of the water-absorbing agent.

Specific examples of the substances include hydrophilic polymers such as starch, starch derivatives, cellulose, cellulose derivatives, polyvinyl alcohol (PVA), polyacrylic acid (salt), and crosslinked products of polyacrylic acid (salt); and compounds such as carbonates, azo compounds, foaming agents that generate various bubbles, surfactants, chelating agents, and chain transfer agents. The addition amount of the hydrophilic polymer, based on the aqueous monomer solution, is preferably 50 mass% or less, more preferably 20 mass% or less, still more preferably 10 mass% or less, and particularly preferably 5 mass% (the lower limit is 0 mass%). Moreover, the addition amount of the compound, based on the aqueous monomer solution, is preferably 5 mass% or less, more preferably 1 mass% or less, and still more preferably 0.5 mass% or less (the lower limit is 0 mass%).

When a water-soluble resin or a water-absorbent resin is used as the hydrophilic polymer, a graft polymer or a water-absorbent resin composition (for example, a starch-acrylic acid (salt) copolymer, a PVA-acrylic acid (salt) copolymer, or the like) is obtained. These graft polymers or water-absorbent resin compositions are also within the scope of the polyacrylic acid (salt)-based water-absorbent resins of the present invention.

Concentration of Monomer Component

The aqueous monomer solution is prepared by selecting, depending upon the purpose, from the various substances (components) described above, and mixing the substances together, as necessary, in such an amount respectively that is stipulated so as to satisfy the above-described ranges. In the present invention, in addition to using the monomers in an aqueous solution, the monomers can also be used in a mixed solution of water and a hydrophilic solvent.

Moreover, from the viewpoint of the physical properties of the water-absorbing agent, the total concentration of the substances (components), which is hereinafter also referred to as "monomer components", is preferably from 10 mass% to 80 mass%, more preferably from 20 mass% to 75 mass%, and still more preferably from 30 mass% to 70 mass%. The concentration of the monomer components is calculated from the following Equation (2).

[Math. 1]

$$\text{Monomer Component Concentration (mass\%)} = \frac{\text{(Monomer Component Mass)}}{\text{(Mass of Aqueous Monomer Solution)}} \times 100 \cdots \text{Equation (2)}$$

In Equation (2), the (Mass of Aqueous Monomer Solution) does not include the mass of the graft component, the water-absorbent resin, or the hydrophobic organic solvent in the reverse phase suspension polymerization.

[2-2] Polymerization Step

The polymerization step is a step of obtaining a crosslinked hydrogel polymer (hereinafter, denoted as a "hydrogel") by polymerizing the aqueous monomer solution that was obtained in the aqueous monomer solution preparation step, the aqueous monomer solution containing the monomer that contains (meth)acrylic acid (salt) as a main component and at least one type of internal crosslinking agent.

Polymerization Initiator

In one embodiment of the present invention, a polymerization initiator is used during polymerization. Examples of the polymerization initiator include thermally decomposable polymerization initiators, photo-decomposable polymerization initiators, or redox-based polymerization initiators that use a reducing agent in combination to accelerate the decomposition of these polymerization initiators. Specific examples of the polymerization initiator include radical polymerization initiators such as sodium persulfate, potassium persulfate, ammonium persulfate, t-butyl hydroperoxide, hydrogen peroxide, and 2,2'-azobis(2-amidinopropane)dihydrochloride. Among these polymerization initiators, at least one type of polymerization initiator is selected in consideration of the form of polymerization and the like. In addition, from the viewpoint of the ease of handling of the polymerization initiator and the physical properties of the water-absorbing agent, at least one of a peroxide and an azo compound is preferably selected as the polymerization initiator. Moreover, when an oxidative radical polymerization initiator is used, redox polymerization may be carried out using a reducing agent such as, for example, sodium sulfite, sodium hydrogen sulfite, iron(II) sulfate, or L-ascorbic acid in combination.

The usage amount of the polymerization initiator based on all the monomers (excluding of course the internal crosslinking agent) is preferably from 0.001 mol% to 1 mol%, more preferably from 0.001 mol% to 0.5 mol%, and even more preferably from 0.01 mol% to 0.1 mol%. The usage amount of the reducing agent based on all the monomers (excluding of course the internal crosslinking agent) is preferably from 0.0001 mol% to 0.02 mol% and more preferably from 0.0005 mol% to 0.015 mol%. By setting the usage amount in this range, a water-absorbing agent having desired water absorption performance is obtained.

In one embodiment of the present invention, the polymerization reaction may be initiated by thermal energy or irradiation with an active energy ray such as a radiation, an electron beam, or an ultraviolet ray. Irradiation with an active energy ray and the polymerization initiator may be used in combination.

Form of Polymerization

Examples of the form of polymerization applicable to the present invention include aqueous solution polymerization, reverse phase suspension polymerization, spray polymerization, droplet polymerization, bulk polymerization, and precipitation polymerization. Among these, from the viewpoints of ease of polymerization control and water absorption performance of the water-absorbing agent, aqueous solution polymerization or reverse phase suspension polymerization is preferably selected, aqueous solution polymerization is more preferably selected, and continuous aqueous solution polymerization is even more preferably selected. The reverse phase suspension polymerization is described in WO 2007/004529 A1, WO 2012/023433 A1, and the like. Moreover, the continuous-type aqueous solution polymerization can be used to produce the water-absorbing agent with high productivity, and specific examples of the continuous-type aqueous solution polymerization described above include continuous belt polymerization described in US 4893999 A, US 6906159 B2, US 7091253 B2, US 7741400 B2, US 8519212 B2, JP 2005-36100 A, and the like, and continuous kneader polymerization described in US 6987151 B2, and the like.

In one embodiment of the aqueous solution polymerization, the temperature of the aqueous monomer solution at the initiation of polymerization is adjusted to a temperature from 10°C to lower than 30°C.

Preferable forms of the continuous aqueous solution polymerization include high-temperature initiation polymerization, high-concentration polymerization, and foam polymerization. Note that high-temperature initiation polymerization is a form of polymerization in which the temperature of the aqueous monomer solution when polymerization is initiated is preferably set to 30°C or higher, more preferably 35°C or higher, even more preferably 40°C or higher, and particularly preferably 50°C or higher (with the upper limit being the boiling point of the aqueous monomer solution). The high-concentration polymerization is a form of polymerization in which the monomer concentration when polymerization is initiated is preferably 30 mass% or greater, more preferably 35 mass% or greater, still more preferably 40 mass% or greater, and particularly preferably 45 mass% or greater (with the upper limit being the saturation concentration of the aqueous monomer solution).

Moreover, each of the above-described forms of polymerization can be carried out in an air atmosphere. However, from the viewpoint of the color tone of the water-absorbing agent, the above-described forms of polymerization are preferably implemented in an atmosphere of an inert gas, such as nitrogen or argon (with the oxygen concentration being 1 vol.% or less). Dissolved oxygen in the aqueous monomer solution is also preferably substituted sufficiently with an inert gas (with the dissolved oxygen being less than 1 mg/L (= ppm)).

[2-3] Gel-Grinding Step

The gel-grinding step is a step of grinding the hydrogel obtained in the polymerization step to thereby obtain a hydrogel having a particulate shape (hereinafter, also referred to as "particulate hydrogel"). To distinguish from "pulverizing" in the pulverization step described later, the present step is referred to as "gel-grinding".

The gel-grinding means that the hydrogel is adjusted to a predetermined size using a gel-grinding apparatus such as a kneader, a meat chopper, or a cutter mill.

As to the details such as the implementation form of the gel-grinding and the operating conditions, the contents described in WO 2011/126079 A1 are also preferably applied to the present invention. Note that when the form of polymerization is kneader polymerization, the polymerization step and the gel-grinding step are performed simultaneously. Moreover, when the particulate hydrogel is obtained in the polymerization step through polymerization such as reverse phase suspension polymerization, spray polymerization, or droplet polymerization, the gel-grinding step is regarded to be carried out simultaneously with the polymerization step. By undergoing the gel-grinding step in the present invention, a water-absorbent resin with an irregularly crushed shape or a water-absorbing agent with an irregularly crushed shape can be obtained.

[2-4] Drying Step

The drying step is a step of producing a dried polymer by drying the hydrogel and/or the particulate hydrogel obtained through the polymerization step and/or the gel-grinding step to a desired resin solid content. The resin solid content is determined from the drying loss (the change in mass after heating 1 g of the water-absorbent resin at 180°C for three hours) and is preferably 80 mass% or more, more preferably from 85 mass% to 99 mass%, further preferably from 90 mass% to 98 mass%, and particularly preferably from 92 mass% to 97 mass%.

Examples of the method for drying the hydrogel and/or the particulate hydrogel include thermal drying, hot air drying, drying under reduced pressure, fluidized bed drying, infrared drying, microwave drying, drum dryer drying, drying by azeotropic dehydration with a hydrophobic organic solvent, and high humidity drying with the use of high-temperature water vapor. Among these, from the viewpoint of drying efficiency, hot air drying is preferable, and band drying in which hot air drying is performed on a ventilation belt is more preferable.

From the viewpoint of the color tone and drying efficiency of the water-absorbent resin and the water-absorbing agent, the drying temperature (temperature of the hot air) in the hot air drying is preferably from 120°C to 250°C, and more preferably from 140°C to 200°C. Drying conditions other than the drying temperature, such as the hot air speed and drying time, may be appropriately set according to the water content percentage and total mass of the particulate hydrogel to be dried and the target resin solid content. When band drying is performed, the various conditions described in WO 2006/100300 A1, WO 2011/025012 A1, WO 2011/025013 A1, WO 2011/111657 A1, and the like, are appropriately applied.

The drying time is preferably from 10 minutes to 2 hours, more preferably from 20 minutes to 150 minutes, and still more preferably from 30 minutes to 100 minutes. By setting the drying temperature and the drying time in the above-described ranges, the physical properties of the resulting water-absorbent resin and the water-absorbing agent can be set within the desired ranges. In addition, the physical properties of the water-absorbent resin as an intermediate product can be set within a desired range.

[2-5] Pulverization Step and Classification Step

The pulverization and classification steps include pulverizing (pulverization step) the dried polymer obtained in the drying step and adjusting the particle size (classification step) to a desired range to obtain a water-absorbent resin powder (base polymer). Through the pulverization step following the drying, a water-absorbent resin or water-absorbing agent in an irregularly crushed shape can be obtained. The pulverization may be carried out twice or more, as necessary.

Examples of the pulverizing apparatus used in the pulverization step include high-speed rotary pulverizing apparatuses such as a roll mill, a hammer mill, a screw mill, and a pin mill; and vibration mills, knuckle type pulverizing apparatuses, and cylindrical mixers. Among these, a roll mill is preferably selected from the viewpoint of pulverizing efficiency. Multiple kinds of these pulverizing apparatuses can be used in combination.

Examples of the method for adjusting the particle size in the classification step include air flow classification and sieve classification using JIS standard sieves (JIS Z 8801-1 (2000)). Among these, the sieve classification is preferably selected from the viewpoint of classification efficiency. Note that the adjustment of the particle size of the water-absorbent resin and water-absorbing agent is not limited to during the pulverization step and/or the classification step, and may be carried out during the polymerization step (particularly, reverse phase suspension polymerization, droplet polymerization, or the like) or during other steps (for example, a granulation step or a fine powder recovery step).

[2-6] Surface-Crosslinking Step

The surface-crosslinking step is a step of providing, on the surface layer of the base polymer obtained through the steps described above, a part having an even higher crosslinking density, and the step includes a mixing step, a heat treatment step, and optionally a cooling step, and the like. In the surface-crosslinking step, radical crosslinking, surface polymerization, a crosslinking reaction with a surface-crosslinking agent, and the like, occur on the surface of the base polymer, and thus a water-absorbent resin (that has been surface-crosslinked) is obtained.

[2-6-1] Mixing Step

The mixing step is a step of obtaining a humidified mixture by mixing a solution containing a surface-crosslinking agent (hereinafter, the solution thereof being referred to as "surface-crosslinking agent solution") with the base polymer in a mixing apparatus.

Surface-Crosslinking Agent

In one embodiment of the present invention, a surface-crosslinking agent is used during the surface-crosslinking. Specific examples of the surface-crosslinking agent include those disclosed in US 7183456 B2. Among these surface-crosslinking agents, at least one surface-crosslinking agent is selected in consideration of reactivity and the like. In addition, from the viewpoint of the ease of handling of the surface-crosslinking agent, the water absorption performance of the water-absorbing agent, and the like, an organic compound in which a covalent bond is formed and that is a surface-crosslinking agent having two or more functional groups reactive with a carboxyl group is preferably selected.

Specific examples of the surface-crosslinking agent include polyhydric alcohol compounds such as ethylene glycol, diethylene glycol, triethylene glycol, tetraethylene glycol, polyethylene glycol, 1,3-propanediol, propylene glycol, 1,4-butanediol, 1,2-butanediol, 1,3-butanediol, 2,3-butanediol, 1,5-pentanediol, 1,4-pentanediol, 1,3-pentanediol, 1,2-pentanediol, 2,3-pentanediol, 2,4-pentanediol, dipropylene glycol, polypropylene glycol, glycerin, polyglycerin, 1,6-hexanediol, 1,5-hexanediol, 1,4-hexanediol, 1,3-hexanediol, 1,2-hexanediol, 2,3-hexanediol, 2,4-hexanediol, diethanolamine, and triethanolamine; polyvalent amine compounds such as ethylenediamine, diethylenetriamine, triethylenetetramine, tetraethylenepentamine, pentaethylenehexamine, polyallylamine, and polyethyleneimine; haloepoxy compounds and condensates of polyvalent amine compounds and haloepoxy compounds; oxazoline compounds such as 1,2-ethylenebisoxazoline; oxazolidinone compounds; alkylene carbonate compounds such as 1,3-dioxolan-2-one(ethylene carbonate), 4-methyl-1,3-dioxolan-2-one, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxopan-2-one; polyvalent glycidyl compounds such as ethylene glycol diglycidyl ether, polyethylene diglycidyl ether, glycerol polyglycidyl ether, diglycerol polyglycidyl ether, polyglycerol polyglycidyl ether, propylene glycol diglycidyl ether, polypropylene glycol diglycidyl ether, and glycidol; oxetane compounds; and vinyl ether compounds. Preferred surface-crosslinking agents include polyhydric alcohol compounds such as propylene glycol and 1,3-propanediol, alkylene carbonate compounds such as ethylene carbonate, polyvalent glycidyl compounds such as ethylene glycol diglycidyl ether, and polyvalent amines such as diethylenetriamine.

The usage amount of the surface-crosslinking agent (the total amount of surface-crosslinking agents when a plurality of types thereof are used) per 100 parts by mass of the base polymer is preferably from 0.01 parts by mass to 10 parts by mass, more preferably from 0.01 parts by mass to 5 parts by mass, still more preferably from 0.01 parts by mass to 2 parts by mass, even more preferably from 0.1 parts by mass to 1.8 parts by mass, and yet even more preferably from 0.5 parts by mass to 1.5 parts by mass. By setting the usage amount of the surface-crosslinking agent in this range, an optimum crosslinked structure can be formed on the surface layer of the base polymer, and a water-absorbent resin and a water-absorbing agent can be obtained with a high level of physical properties. In addition, adjusting the amount of the surface-crosslinking agent according to the type is also an effective measure for optimizing the gel bulk density of the water-absorbent resin or the water-absorbing agent.

The surface-crosslinking agent is preferably added to the base polymer in the form of an aqueous solution. In this case, the usage amount of water per 100 parts by mass of the base polymer is preferably from 0.1 parts by mass to 20 parts by mass, more preferably from 0.3 parts by mass to 15 parts by mass, and still more preferably from 0.5 parts by mass to 10 parts by mass. By setting the usage amount of water in this range, the ease of handling of the surface-crosslinking agent solution is improved, and the surface-crosslinking agent can be uniformly mixed with the base polymer.

The surface-crosslinking agent solution can also be obtained by using a hydrophilic organic solvent in combination with the water as necessary. In this case, the usage amount of the hydrophilic organic solvent is preferably small so as not to cause an unpleasant odor, and per 100 parts by mass of the base polymer, the usage amount of the hydrophilic organic solvent is preferably 5 parts by mass or less, more preferably 3 parts by mass or less, still more preferably 2 parts by mass or less, and yet even more preferably 1 part by mass or less. However, from the viewpoint of carrying out appropriate surface-crosslinking, a hydrophilic organic solvent may be added, and the addition amount thereof is preferably 0.1 parts by mass or more, and more preferably 0.5 parts by mass or more, per 100 parts by mass of the base polymer.

Specific examples of the hydrophilic organic solvent include lower alcohols (for example, alcohols having from 1 to 3 carbons) such as methyl alcohol and isopropyl alcohol; ketones such as acetone; ethers such as dioxane; amides such as N,N-dimethylformamide; and sulfoxides such as dimethyl sulfoxide.

Mixing Method and Mixing Conditions

To mix the base polymer and the surface-crosslinking agent solution, a method is selected in which the surface-crosslinking agent solution is prepared in advance, and the solution is preferably sprayed or dropped onto the base polymer, and more preferably sprayed and mixed.

[2-6-2] Heat Treatment Step

The heat treatment step is a step of applying heat to the humidified mixture obtained in the mixing step to cause a crosslinking reaction on the surface of the base polymer.

In the heat treatment of the humidified mixture, the humidified mixture may be heated in a stationary state or may be heated in a flowing state using power such as stirring, but it is preferable to heat the humidified mixture under stirring in that the entire humidified mixture can be uniformly heated. From the above-described viewpoint, examples of the heat treatment apparatus that performs the heat treatment include a paddle dryer, a multi-fin processor, and a tower dryer.

From viewpoints such as the type and amount of the surface-crosslinking agent, and the water absorption performance of the water-absorbing agent, the heating temperature in this step is preferably from 150°C to 250°C, more preferably from 170°C to 250°C, and even more preferably from 180°C to 230°C. The heating time is at least 5 minutes, and is preferably at least 7 minutes. The upper limit of the heating time is preferably 150 minutes or less, more preferably 120 minutes or less, and further preferably 100 minutes or less. Controlling the heating temperature and the heating time to within the above-described ranges improves the water absorption performance of the obtained water-absorbing agent, and thus is preferable.

[2-6-3] Cooling Step

The cooling step is an optional step provided, as necessary, after the heat treatment step. The present step is a step of forcibly cooling the high-temperature water-absorbent resin that has been subjected to the heat treatment step to a predetermined temperature to rapidly terminate the surface-crosslinking reaction.

[2-7] Chelating Agent Addition Step

The water-absorbent resin composition of one aspect of the present invention contains a chelating agent. The chelating agent may be added to the monomer, the hydrogel, the dried polymer, the base polymer (water-absorbent resin powder), or the surface-crosslinked water-absorbent resin. In one embodiment of the present invention, the chelating agent is added after the polymerization step, after the gel-grinding step, after the pulverization step and the classification step, or after the surface-crosslinking step. In particular, the crosslinking agent is preferably added after the surface-crosslinking step. After the surface-crosslinking step, the chelating agent may be added simultaneously with or sequentially to the copper silicate.

In one embodiment of the present invention, the usage amount of the chelating agent in terms of the active ingredient is from 10 to 1000 ppm by mass, from 20 to 800 ppm by mass, from 30 to 600 ppm by mass, from 40 to 500 ppm by mass, from 50 to 300 ppm by mass, from 60 to 200 ppm by mass, or from 70 to 150 ppm by mass, relative to the water-absorbent resin.

In one embodiment of the present invention, a heating treatment may be carried out after the addition of the chelating agent. The temperature in the heating treatment is, for example, preferably from 45 to 75°C, from 50 to 70°C, or from 55 to 65°C. The heating treatment time may vary depending on the heating temperature, but the approximate heating treatment time is preferably, for example, from 5 to 60 minutes, from 10 to 50 minutes, or from 15 to 40 minutes. In one embodiment of the present invention, the heating treatment may be carried out after the addition of the chelating agent, after which the copper silicate may be added.

[2-8] Copper Silicate Addition Step

The water-absorbent resin composition according to one aspect of the present invention contains a copper silicate. The copper silicate may be added to the monomer, the hydrogel, the dried polymer, the base polymer (water-absorbent resin powder), or the surface-crosslinked water-absorbent resin. In one embodiment of the present invention, the copper silicate is added after the polymerization step, after the gel-grinding step, after the pulverization step and the classification step, or after the surface-crosslinking step. In particular, the copper silicate is preferably added after the surface-crosslinking step. In this manner, the existence ratio of the copper silicate is higher on the surface of the water-absorbent resin than inside the water-absorbent resin. Therefore, in one embodiment of the present invention, a water-absorbent resin composition can be provided in which the existence ratio of copper silicate is higher on the surface of the water-absorbent resin than inside the water-absorbent resin.

In one embodiment of the present invention, the usage amount of the copper silicate relative to the water-absorbent resin is from 0.01 to 10 mass%, from 0.05 to 9 mass%, from 0.1 to 8 mass%, from 0.2 to 7 mass%, from 0.3 to 6 mass%, from 0.4 to 5 mass%, from 0.5 to 4 mass%, from 0.6 to 3 mass%, or from 0.7 to 2 mass%.

In one embodiment of the present invention, no intentional heating treatment is applied after the addition of the copper silicate.

[2-9] Step of Adding Coloring Inhibitor or Urine Resistance Improver

In general, the water-absorbent resin tends to easily discolor or deteriorate, and therefore, in the present invention, a coloring inhibitor or a urine resistance (weather resistance) improver selected from inorganic or organic reducing agents (particularly, sulfur-based inorganic reducing agents) may be further contained in order to suppress discoloration or deterioration. The usage amounts of the coloring inhibitor and urine resistance improver in terms of solid content are preferably from 0 to 3 parts by mass, more preferably from 0.001 to 1 parts by mass, and particularly preferably from 0.05 to 0.5 parts by mass, per 100 parts by mass of the water-absorbent resin. Such a preferable usage amount coincides with the preferable content in the water-absorbent resin composition. That is, according to one embodiment of the present invention, the content of the coloring inhibitor and the content of the urine resistance improver in the water-absorbent resin composition are each independently preferably from 0 to 3 parts by mass, more preferably from 0.001 to 1 part by mass, and particularly preferably from 0.05 to 0.5 parts by mass, per 100 parts by mass of the water-absorbent resin. The coloring inhibitor and the urine resistance improver are added to the monomer, the hydrogel, the dried polymer, the powder, or the like, and the addition step is appropriately determined after the polymerization step, but among these, the reducing agent is consumed by polymerization, and therefore the reducing agent is preferably added after polymerization, more preferably after drying, and particularly preferably after surface-crosslinking.

Examples of the inorganic or organic reducing agent (particularly, a sulfur-based inorganic reducing agent) that can be used include sulfur-based reducing agents described in US 2010/0062252 A1 and the like, and particularly sulfites or hydrogen sulfites.

[2-10] Other Steps

In the present invention, in addition to the above-described steps, a granulating step, a particle size adjustment step, a fine powder removal step, a fine powder recovery step, a step of reusing fine powder, a step of adding other additives, an iron removal step, and the like, can be carried out as necessary. At least one step selected from among a transportation step, a stocking step, a packaging step, a storage step, and the like may be further included.

The particle size adjustment step includes a step of classifying and removing fine powder after the surface-crosslinking step, and/or a step of classifying and pulverizing the water-absorbent resin when the water-absorbent resin is aggregated and exceeds a desired size. The step of reusing the fine powder includes a step of adding the fine powder as is or in the form of a large hydrogel obtained in the granulation step to the hydrogel serving as the raw material in any step of the process of producing a water-absorbent resin.

In the step of adding the coloring inhibitor or the urine resistance improver, in order to impart various functions to the water-absorbing agent, one or more other additives selected from an oxidizing agent, an organic powder such as a metal soap, a pulp, thermoplastic fibers, and the like can be added together with the coloring inhibitor or the urine resistance improver or in place of the coloring inhibitor or the urine resistance improver. These other additives may be mixed simultaneously with or separately from the surface-crosslinking agent. That is, the water-absorbent resin composition of the present invention may contain such other additives.

[3] Properties of Water-Absorbent Resin Composition

[3-1] Other Properties of Water-Absorbent Resin Composition

In one embodiment of the present invention, the water-absorbent resin composition preferably further exhibits the following properties.

(3-1-1) Mass-Average Particle Size (D50)

In one embodiment of the present invention, the mass-average particle size (D50) of the water-absorbent resin composition is preferably 300 $\mu$m or greater, more preferably 305 $\mu$m or greater, even more preferably 310 $\mu$m or greater, and particularly preferably 315 $\mu$m or greater. Moreover, the mass-average particle size (D50) thereof is preferably 600 $\mu$m or less, more preferably 550 $\mu$m or less, still more preferably 500 $\mu$m or less, and particularly preferably 450 $\mu$m or less. The range of the mass-average particle size (D50) may be any combination of the above-described upper and lower limit values.

By setting the mass-average particle size (D50) of the water-absorbent resin composition to within the above-described range, in one embodiment of the present invention described below, the centrifuge retention capacity (CRC) and the absorption against pressure (AAP) of the water-absorbent resin composition can be controlled in a well-balanced manner. That is, by setting the mass-average particle size (D50) thereof to within the above-described range, the centrifuge retention capacity (CRC) and the absorption against pressure (AAP) can be increased, and when the particle roughness of the water-absorbent resin is suppressed and the water-absorbent resin is used in an absorbent article such as a disposable diaper or a sanitary napkin, the texture and the wearing feeling can be improved.

(3-1-2) Centrifuge Retention Capacity (CRC)

In one embodiment of the present invention, centrifuge retention capacity (CRC) of the water-absorbent resin composition is usually 5 g/g or higher, preferably 20 g/g or higher, more preferably 24 g/g or higher, and still more preferably 30 g/g or higher. The upper limit thereof is not particularly limited, and a higher CRC value is preferable. However, from the viewpoint of achieving good balance with other physical properties, the CRC upper limit is preferably 70 g/g or less, more preferably 50 g/g or less, and still more preferably 45 g/g or less. Therefore, as a typical range of the centrifuge retention capacity (CRC), the CRC value can be appropriately selected within the range of the upper and lower limit values described above. For example, any range such as from 5 to 70 g/g, from 20 to 50 *g/g,* or from 24 to 45 g/g can be selected.

When the CRC value is less than 5 *g/g,* the water absorption amount of the water-absorbent resin composition is small, and the water-absorbent resin composition is not suitable as an absorbent body of an absorbent article such as a disposable diaper. Moreover, when the CRC value exceeds 70 g/g, the rate at which body fluids such as urine and blood are absorbed is reduced, and therefore the water-absorbent resin composition is not suitable for use in a high water absorption rate-type disposable diapers and the like. The CRC value can be controlled by an internal crosslinking agent, a surface-crosslinking agent, or the like.

(3-1-3) Absorption Against Pressure (AAP)

In one embodiment of the present invention, the absorption against pressure (AAP) of the water-absorbent resin composition is preferably 5 g/g or greater, more preferably 8 g/g or greater, still more preferably 10 g/g or greater, yet even more preferably 12 g/g or greater, preferably 14 g/g or greater, still further preferably 18 g/g or greater, even more preferably 22 g/g or greater, yet even more preferably 25 g/g or greater, still more preferably 28 g/g or greater, and even more preferably 30 g/g or greater. The upper limit value is not particularly limited, but is preferably 40 g/g or less, 35 g/g or less, or 33 g/g or less.

When the AAP is less than 5 g/g and the water-absorbent resin composition is actually used in a disposable diaper or the like, the absorption amount in a state in which pressure is applied to the absorbent body decreases, and therefore such a water-absorbent resin composition is not suitable for use as an absorbent body of an absorbent article such as a disposable diaper. The AAP can be controlled by the particle size, the surface-crosslinking agent, or the like.

[4] Applications for Water-Absorbent Resin Composition (Absorbent Body, Absorbent Layer)

The water-absorbent resin composition according to the present invention is preferably used mainly as an absorbent body (absorbent layer) of an absorbent article such as a disposable diaper or a sanitary napkin, and is more preferably used as an absorbent body (absorbent layer) of an absorbent article in which the amount of use per one absorbent article is large. Therefore, in one embodiment of the present invention, an absorbent body including the water-absorbent resin composition and hydrophilic fibers is provided.

The term absorbent body means an article obtained by molding the water-absorbent resin composition into a sheet shape, a fibrous shape, a cylindrical shape, or the like, and is preferably molded into a sheet shape to form an absorbent layer. In addition to the water-absorbent resin composition according to the present invention, an absorbent material such as pulp fibers, or an adhesive, a nonwoven fabric, or the like can be molded and used in combination. In this case, the amount of the water-absorbing agent in the absorbent body (absorbent layer) (hereinafter, the amount thereof is referred to as the "core concentration") is preferably in a range of from 20 to 100 mass%, more preferably in a range of from 30 to 90 mass%, and still more preferably in a range of from 40 to 80 mass%. When the core concentration is less than 20 mass%, the usage amount of the water-absorbent resin composition is small, and for example, deodorizing performance may not be sufficiently imparted to the entire diaper, which is not preferable.

When the absorbent body according to the present invention is produced from the water-absorbent resin composition and hydrophilic fibers, the production method is not particularly limited, and examples thereof include a method of producing the absorbent body by dry-mixing the water-absorbent resin composition and the hydrophilic fibers at a ratio to achieve the above-described core concentration using a mixing apparatus such as a mixer, then forming the obtained mixture into a web shape by, for example, air-based paper-making, and then compression molding as necessary. Such an absorbent body is compression-molded to a density of, for example, from 0.001 to 0.50 g/cc and a basis weight of from 0.01 to 0.20 g/cm$^2$.

[5] Absorbent Article

The absorbent article according to the present invention contains the absorbent body (absorbent layer) and is usually provided with a liquid-permeable top sheet and a liquid-impermeable back sheet. Examples of the absorbent article include a disposable diaper and a sanitary napkin. Accordingly, in one embodiment of the present invention, an absorbent article is provided that includes an absorbent body, a top sheet having liquid permeability, and a back sheet having liquid impermeability.

In a case in which the absorbent article is, for example, a disposable diaper, the disposable diaper is produced by sandwiching an absorbent body containing the water-absorbent resin composition of one embodiment of the present invention between a liquid-permeable top sheet located on the side that comes into contact with the skin of a person when the disposable diaper is worn and a liquid-impermeable back sheet located on the outer side when the disposable diaper is worn. The disposable diaper is further provided with a member known to a person skilled in the art, such as an adhesive tape for fixing the disposable diaper while being worn.

In addition to use in disposable diapers and sanitary napkins, the water-absorbent resin composition according to the present invention can also be suitably used in applications for pets and portable toilets.

The present invention includes the following aspects and forms.

1. A water-absorbent resin composition, including a water-absorbent resin, a copper silicate, and a chelating agent.

2. The water-absorbent resin composition according to 1., wherein the chelating agent includes at least one compound selected from the group consisting of an amino polycarboxylic acid, an amino polyphosphoric acid, and salts thereof.

3. The water-absorbent resin composition according to 1. or 2., wherein an existence ratio of the copper silicate is higher on a surface of the water-absorbent resin than inside the water-absorbent resin.

4. The water-absorbent resin composition according to any of 1. to 3., wherein the copper silicate has an amorphous structure.

5. The water-absorbent resin composition according to any of 1. to 4., wherein a solubility of the copper silicate in ion-exchanged water at 25°C is 0.5 g/100 mL-$H_2O$ or less.

6. The water-absorbent resin composition according to any of 1. to 5., wherein the copper silicate contains a sulfur atom.

7. The water-absorbent resin composition according to any of 1. to 6., wherein, of all polyvalent metal components contained in the copper silicate, copper accounts for 50 mass% or more.

8. The water-absorbent resin composition according to any of 1. to 7., wherein, when the copper silicate contains zinc, a mass ratio of a content of zinc to a content of silicon in the copper silicate is less than 50/50.

9. An absorbent body, including the water-absorbent resin composition described in any of 1. to 8.

10. An absorbent article, including the absorbent body described in aspect 9, a liquid-permeable top sheet, and a liquid-impermeable back sheet.

Examples

The present invention will be described more specifically below through examples and comparative examples, but the present invention is not limited to these examples and comparative examples. Moreover, examples obtained by appropriately combining the technical means disclosed in the various examples are also included in the scope of the present invention. Unless otherwise noted, the electrical devices used in the examples and comparative examples to measure the physical properties of the water-absorbent resins and water-absorbent resin compositions use a power supply of 200 V or 100 V, and 60 Hz. In addition, unless otherwise noted, the physical properties of the water-absorbent resins and the water-absorbent resin compositions were measured under conditions of room temperature (from 20°C to 25°C) and a relative humidity of 50 ±5% RH. In addition, for simplicity, the unit of "liter" may be denoted as "1" or "L", and "mass%" may be denoted as "wt.%".

Production Example 1

An aqueous monomer solution was prepared by mixing 67.0 parts by mass of a 37% aqueous sodium acrylate solution, 10.2 parts by mass of acrylic acid, 0.084 parts by mass of polyethylene glycol diacrylate (average number of added moles of ethylene oxide: 9), and 22.0 parts by mass of water. Nitrogen gas was blown into the aqueous monomer solution in a vat to reduce the amount of oxygen dissolved in the solution to 0.1 ppm or less. Subsequently, the temperature of the aqueous monomer solution was adjusted to 18°C in a nitrogen atmosphere, and then 0.16 parts by mass of a 5 mass% aqueous sodium persulfate solution, 0.16 parts by mass of a 5 mass% aqueous 2,2'-azobis(2-amidinopropane) dihydrochloride solution, 0.15 parts by mass of a 0.5 mass% aqueous L-ascorbic acid solution, and 0.17 parts by mass of a 0.35 mass% aqueous hydrogen peroxide solution were added dropwise in this order with stirring. Polymerization began immediately after the dropwise addition of the hydrogen peroxide. The stirring was then stopped, and after 10 minutes the temperature of the monomer reached a peak temperature. The peak temperature was 85°C. Subsequently, the vat was immersed in a hot water bath at 80°C and aged for 10 minutes, and the resulting transparent crosslinked hydrogel polymer was crushed with a meat chopper. Next, this finely divided crosslinked hydrogel polymer (particulate hydrogel) was spread on a 50-mesh (opening size of 300 μm) wire net and dried with hot air at 180°C for 30 minutes, and thereby a dried polymer was obtained.

The obtained dried polymer was pulverized with a pulverizing apparatus, and classified into a polymer that passed through a sieve of 500 μm and remained on a sieve of 105 μm, and thereby a water-absorbent resin powder was obtained.

An aqueous surface-crosslinking agent solution containing 0.05 parts by mass of ethylene glycol diglycidyl ether, 1 part by mass of propylene glycol, 3 parts by mass of water, and 1 part by mass of isopropyl alcohol was mixed with 100 parts by mass of the obtained water-absorbent resin powder. The mixture was subjected to heating-treatment at 180°C for 40 minutes, and surface-crosslinked water-absorbent resin particles were thereby obtained.

Example 1

An amount of 1.0 parts by mass of a 1.0 mass% diethylenetriamine pentaacetic acid trisodium aqueous solution was added to 100 parts by mass of the surface-crosslinked water-absorbent resin particles obtained in Production Example 1 and mixed, and the mixture was heated at 60°C for 30 minutes. Subsequently, 1.0 parts by mass of a copper silicate (A) (volume-average particle size: 7 $\mu$m, solubility in ion-exchanged water at 25°C: 0 g/100 mL-$H_2O$) satisfying the atomic ratio to Si and the weight ratio to Si presented in Table 1 below was added thereto and mixed, and a mixture was obtained. Subsequently, the mixture was passed through a JIS standard sieve with an opening of 850 $\mu$m, and a water-absorbent resin composition (1) was thereby obtained. Measurement results for various physical property values of the obtained water-absorbent resin composition (1) are presented in Table 5. The copper silicate (A) had an amorphous structure.

The atomic ratio and the weight ratio were measured by SEM-EDS analysis using a scanning electron microscope. Specifically, an image of the additive was obtained at a magnification of 1000 times using the JCM-6000 Neoscope Benchtop Scanning Electron Microscope (available from JEOL Ltd.), and the atomic ratio and the weight ratio of the constituent elements of the additive were analyzed by energy dispersive X-ray spectroscopy (EDS).

Example 2

A water-absorbent resin composition (2) was obtained in the same manner as in Example 1 with the exception that the 1.0 mass% diethylenetriamine pentaacetic acid trisodium aqueous solution was changed to a 1.0 mass% ethylenediamine tetra(methylenephosphonic acid) pentasodium aqueous solution. The measurement results of the various physical property values of the water-absorbent resin composition (2) are presented in Table 5.

Example 3

A water-absorbent resin composition (3) was obtained in the same manner as in Example 1 with the exception that the copper silicate (A) was changed to a copper silicate (B) (volume-average particle size: 3 $\mu$m, solubility in ion-exchanged water at 25°C: 0 g/100 mL-$H_2O$) in which the atomic ratio to Si and the weight ratio to Si were as indicated in the following table. The measurement results of the various physical property values of the water-absorbent resin composition (3) are presented in Table 5. The copper silicate (B) had an amorphous structure.

[Table 1]

| <Atomic Ratio to Si> | | | | | | |
|---|---|---|---|---|---|---|
| | Si | Cu | S | O | Na | Al |
| Copper silicate (A) | 100 | 5.55 | 6.10 | 535.95 | 23.75 | - |
| Copper silicate (B) | 100 | 38.83 | 0.13 | 706.77 | - | 2.17 |

| <Weight Ratio to Si> | | | | | | |
|---|---|---|---|---|---|---|
| | Si | Cu | S | O | Na | Al |
| Copper silicate (A) | 100 | 12.55 | 6.96 | 305.31 | 19.44 | - |
| Copper silicate (B) | 100 | 87.85 | 0.15 | 402.62 | - | 2.09 |

Comparative Example 1

A comparative water-absorbent resin (1) was obtained in the same manner as in Example 1 with the exception that the 1.0 parts by mass of the 1.0 mass% diethylenetriamine pentaacetic acid trisodium aqueous solution was changed to 1.0 parts by mass of water, and the copper silicate (A) was not added. The measurement results of the various physical property values of the comparative water-absorbent resin (1) are presented in Table 5.

Comparative Example 2

A comparative water-absorbent resin composition (2) was obtained in the same manner as in Example 1 with the exception that the copper silicate (A) was not added. The measurement results of the various physical property values of the comparative water-absorbent resin composition (2) are presented in Table 5.

Comparative Example 3

A comparative water-absorbent resin composition (3) was obtained in the same manner as in Example 1 with the exception that a chelating agent was not added. The measurement results of the various physical property values of the comparative water-absorbent resin composition (3) are presented in Table 5.

Comparative Example 4

A comparative water-absorbent resin composition (4) was obtained in the same manner as in Example 1 with the exception that the copper silicate (A) was changed to a silicate (volume-average particle size: 3.5 $\mu$m, solubility in ion-exchanged water at 25°C: 0 g/100 mL-$H_2O$) containing no copper and satisfying the atomic ratio to Si and the weight ratio to Si presented in Table 2 below. The measurement results of the various physical property values of the comparative water-absorbent resin composition (4) are presented in Table 5.

[Table 2]

| <Atomic Ratio to Si> | | | | | | | |
|---|---|---|---|---|---|---|---|
| | Si | Cu | S | O | Na | Al | Zn |
| Copper-free silicate | 100 | - | 2.74 | 883.73 | 40.95 | 24.50 | 55.94 |

| <Weight Ratio to Si> | | | | | | | |
|---|---|---|---|---|---|---|---|
| | Si | Cu | S | O | Na | Al | Zn |
| Copper-free silicate | 100 | - | 3.13 | 503.42 | 33.52 | 23.53 | 130.23 |

Comparative Example 5

A comparative water-absorbent resin composition (5) was obtained in the same manner as in Example 1 with the exception that the copper silicate (A) was changed to copper(II) gluconate (solubility in ion-exchanged water at 25°C: 30 g/100 mL-$H_2O$). The measurement results of the various physical property values of the comparative water-absorbent resin composition (5) are presented in Table 5.

Comparative Example 6

A comparative water-absorbent resin composition (6) was obtained in the same manner as in Example 1 with the exception that the copper silicate (A) was changed to sodium copper chlorophyllin (solubility in ion-exchanged water at 25°C: 1 g/100 mL-$H_2O$). The measurement results of the various physical property values of the comparative water-absorbent resin composition (6) are presented in Table 5.

Comparative Example 7

A comparative water-absorbent resin composition (7) was obtained in the same manner as in Example 1 with the exception that the copper silicate (A) was changed to a copper oxide-zinc oxide composite aluminosilicate (zeolite supporting copper and zinc) (trade name: Dashlite CZU, available from Sinanen Zeomic Co., Ltd., average particle size = 4 $\mu$m) satisfying the atomic ratio to Si and the weight ratio to Si presented in Table 3 below. The measurement results of the various physical property values of the comparative water-absorbent resin composition (7) are presented in Table 5.

[Table 3]

| <Atomic Ratio to Si> | | | | | | | |
|---|---|---|---|---|---|---|---|
| | Si | Cu | S | O | Na | Al | Zn |
| Copper oxide-zinc oxide composite aluminosilicate | 100 | 2.16 | - | 569.32 | - | 2.27 | 1.02 |

| <Weight Ratio to Si> | | | | | | | |
|---|---|---|---|---|---|---|---|
| | Si | Cu | S | O | Na | Al | Zn |
| Copper oxide-zinc oxide composite aluminosilicate | 100 | 4.89 | - | 324.31 | - | 2.18 | 2.38 |

Evaluation of Water-Absorbent Resin Composition

(a) Mass-Average Particle Size (D50)

The mass-average particle size (D50) of the water-absorbent resin composition was measured in accordance with the EDANA WSP 220.2 particle size analysis method using a combination of sieves having mesh sizes of 850 $\mu$m, 710 $\mu$m, 600 $\mu$m, 500 $\mu$m, 300 $\mu$m, 150 $\mu$m, and 45 $\mu$m in this order from the top.

(b) Centrifuge Retention Capacity (CRC)

The CRC of the water-absorbent resin composition was measured in accordance with the EDANA WSP 241.3(10) method.

(c) Absorption Against Pressure (AAP 0.3 psi)

The AAP of the water-absorbent resin composition was measured in accordance with the EDANA NWSP 242.0.R2(15) method. Evaluation was made with a load of 0.3 psi (= 21 g/cm$^2$ = 2.06 kPa).

(d) Human Urine Deodorization Performance Evaluation

A simple absorbent body was prepared by mixing 2.0 g of a water-absorbent resin (or water-absorbent resin composition) obtained in the examples and comparative examples described above with 4.0 g of ground wood pulp. The simple absorbent body was placed in a polypropylene cup having a lid and a volume of 250 ml, and 50 ml of human urine collected from a plurality of adults was inserted therein. Human urine within 2 hours of excretion was used. The container was covered with the lid, and the simple absorbent body was maintained at 40°C. The lid was opened 3 hours after the liquid was absorbed, and a panel of 20 adults smelled the odor at a position of approximately 3 cm from the top of the cup to determine the deodorizing effect. Each panelist issued a score for the deodorizing effect according to the following criteria based on a 6-point scale, and an average value of the scores was calculated and used as the determined deodorizing effect.

[Table 4]

| Evaluation | Determination Criteria |
|---|---|
| 5 | Very strong urine odor |
| 4 | Strong urine odor |
| 3 | Weak urine odor |
| 2 | Slight urine odor is perceived. |
| 1 | Slight odor not recognizable as urine odor |
| 0 | Odorless |

(e) Methyl Mercaptan (MM) Concentration

1. An amount of 5 g of the water-absorbent resin composition was placed in a mayonnaise jar (available from Nihon Yamamura Glass Co., Ltd., outer diameter: 62 mm, height: 109.2 mm, volume: 241.8 mL), and 25 g of a 0.9 mass% aqueous sodium chloride solution was added thereto to swell the water-absorbent resin composition.
2. The mayonnaise jar was covered with a lid and placed in a paint shaker (No. 488 test disperser, available from Toyo Seiki Seisaku-sho, Ltd.). Next, the paint shaker was shaken at 800 (cycles/min) for 1 minute and then stopped.
3. To the mayonnaise jar, 1 g of a 0.1 mass% aqueous sodium methyl mercaptan solution (a commercially available reagent of a 15% aqueous solution diluted with a 0.9 mass% aqueous sodium chloride solution) was added.

4. The mayonnaise jar was covered with the lid and stored at 30°C for 1 hour, after which the lid was opened, and the concentration of methyl mercaptan in the air above the bottle was measured with a detection tube.

(f) Methyl Mercaptan (MM) Concentration Increase Rate

The same operation as in 4. described above was carried out with the exception that the storage temperature was changed to 40°C, and the methyl mercaptan (MM) concentration at 40°C was measured. The MM concentration increase rate due to the increase in temperature was calculated according to the following equation.

Increase rate [%] of MM concentration due to temperature increase = {(MM concentration at 40°C)/(MM concentration at 30°C) - 1} $\times$ 100

[Table 5]

| | Additive | | | | Water-absorbent resin composition | | | | | | |
|---|---|---|---|---|---|---|---|---|---|---|---|
| | Copper compound | | Chelating agent | | | | | | | | |
| | Type | Addition amount [mass%] | Type | Addition amount [ppm] | CRC [g/g] | AAP 0.3 psi [g/g] | D50 [μm] | Deodorizing performance evaluation | MM Concentration (stored at 30°C) [ppm] | MM Concentration (stored at 40°C) [ppm] | MM Concentration increase rate [%] |
| Example 1 | Copper silicate (A) | 1.0 | Diethylenetriamine pentaacetic acid trisodium | 100 | 33.4 | 31.5 | 378 | 2.0 | 15 | 15 | 0 |
| Example 2 | Copper silicate (A) | 1.0 | Ethylenediamine tetra(methylenephosphonic acid) pentasodium | 100 | 33.4 | 31.5 | 377 | 2.0 | 15 | 15 | 0 |
| Example 3 | Copper silicate (B) | 1.0 | Diethylenetriamine pentaacetic acid trisodium | 100 | 33.5 | 31.6 | 378 | 2.0 | 15 | 15 | 0 |
| Comparative Example 1 | - | - | - | - | 34.0 | 32.0 | 380 | 4.0 | 70 | | |
| Comparative Example 2 | - | - | Diethylenetriamine pentaacetic acid trisodium | 100 | 33.7 | 31.8 | 383 | 4.0 | 70 | 70 | 0 |
| Comparative Example 3 | Copper silicate (A) | 1.0 | - | - | 33.7 | 31.7 | 378 | 2.0 | 25 | | |
| Comparative Example 4 | Copper-free silicate | 1.0 | Diethylenetriamine pentaacetic acid trisodium | 100 | 33.5 | 31.5 | 383 | 4.0 | 70 | | |
| Comparative Example 5 | Copper(II) gluconate | 1.0 | Diethylenetriamine pentaacetic acid trisodium | 100 | 33.5 | 31.6 | 377 | 3.5 | 35 | | |
| Comparative Example 6 | Sodium copper chlorophyllin | 1.0 | Diethylenetriamine pentaacetic acid trisodium | 100 | 33.3 | 31.7 | 382 | 3.5 | 40 | | |
| Comparative Example 7 | Copper oxide-zinc oxide composite aluminosilicate | 1.0 | Diethylenetriamine pentaacetic acid trisodium | 100 | 33.4 | 31.7 | 382 | 3.0 | 25 | 40 | 60 |

Discussion

Comparative Example 1 is a blank test using only a water-absorbent resin, and neither could the urine odor nor the methyl mercaptan odor be suppressed.

From a comparison of Comparative Examples 1 and 2, it was found that the effect of suppressing urine odor is not exhibited by merely applying the chelating agent to the water-absorbent resin. Meanwhile, as demonstrated in Examples 1 to 3, it was found that both urine odor and methyl mercaptan odor can be suppressed by combining and applying a chelating agent and a copper silicate to the water-absorbent resin. Moreover, it should be noted that the water-absorbent resin compositions of Examples 1 to 3 successfully suppressed the recurrence of the methyl mercaptan odor even when the storage environment was changed to a high temperature environment.

As demonstrated in Comparative Example 4, even when a silicate containing no copper is applied to the water-absorbent resin, neither the urine odor nor the methyl mercaptan odor can be suppressed.

Moreover, as demonstrated in Comparative Examples 5 and 6, even when a water-soluble copper compound that is not a silicate is applied to a water-absorbent resin, neither the urine odor nor the methyl mercaptan odor can be suppressed.

As demonstrated in Comparative Example 7, when an inorganic antibacterial agent in which an antibacterial metal such as copper or zinc is supported on an inorganic compound is applied to a water-absorbent resin, the urine odor and the methyl mercaptan odor can be suppressed to some extent, but when the storage environment changes to a high temperature, the methyl mercaptan odor that was suppressed recurs once again.

Thus, it was found that by applying a copper silicate and a chelating agent in combination to a water-absorbent resin, an effect of further improving the reduction of urine odor as compared with the conventional technology and suppressing the recurrence of bod odors in a high temperature environment can be obtained.

The present application is based on Japanese Patent Application No. 2023-143714, filed on September 5, 2023, the entire disclosure contents of which is incorporated herein by reference.

**Claims**

1. A water-absorbent resin composition, comprising a water-absorbent resin, a copper silicate, and a chelating agent.

2. The water-absorbent resin composition according to claim 1, wherein the chelating agent comprises at least one compound selected from the group consisting of an amino polycarboxylic acid, an amino polyphosphoric acid, and salts thereof.

3. The water-absorbent resin composition according to claim 1 or 2, wherein an existence ratio of the copper silicate is higher on a surface of the water-absorbent resin than inside the water-absorbent resin.

4. The water-absorbent resin composition according to any one of claims 1 to 3, wherein the copper silicate has an amorphous structure.

5. The water-absorbent resin composition according to any one of claims 1 to 4, wherein a solubility of the copper silicate in ion-exchanged water at 25°C is 0.5 g/100 mL-$H_2O$ or less.

6. The water-absorbent resin composition according to any one of claims 1 to 5, wherein the copper silicate comprises a sulfur atom.

7. The water-absorbent resin composition according to any one of claims 1 to 6, wherein, of all polyvalent metal components contained in the copper silicate, copper accounts for 50 mass% or more.

8. An absorbent body, comprising the water-absorbent resin composition described in any one of claims 1 to 7 and a hydrophilic fiber.

9. An absorbent article, comprising the absorbent body described in claim 8, a liquid-permeable top sheet, and a liquid-impermeable back sheet.

## INTERNATIONAL SEARCH REPORT

International application No.

**PCT/JP2024/029757**

### A. CLASSIFICATION OF SUBJECT MATTER

*C08L 101/14*(2006.01)i; *A61F 13/15*(2006.01)i; *A61F 13/53*(2006.01)i; *B01J 20/26*(2006.01)i; *C08J 3/20*(2006.01)i; *C08K 3/34*(2006.01)i; *C08K 5/00*(2006.01)i; *C08K 5/49*(2006.01)i; *C08K 5/092*(2006.01)i
FI: C08L101/14; B01J20/26 D; A61F13/53 300; A61F13/15 141; C08K3/34; C08K5/00; C08K5/092; C08K5/49; C08J3/20 Z CEY

According to International Patent Classification (IPC) or to both national classification and IPC

### B. FIELDS SEARCHED

Minimum documentation searched (classification system followed by classification symbols)

C08L101/14; A61F13/15; A61F13/53; B01J20/26; C08J3/20; C08K3/34; C08K5/00; C08K5/49; C08K5/092

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2007-20931 A (KAISUI KAGAKU KENKYUSHO KK) 01 February 2007 (2007-02-01) | 1-9 |
| A | JP 2019-1154 A (RICOH COMPANY, LTD.) 10 January 2019 (2019-01-10) | 1-9 |
| A | WO 2021/200137 A1 (SUMITOMO SEIKA CHEMICALS CO., LTD.) 07 October 2021 (2021-10-07) | 1-9 |

☐ Further documents are listed in the continuation of Box C.    ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **08 October 2024** | **22 October 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**
Information on patent family members

International application No.

**PCT/JP2024/029757**

| Patent document cited in search report | | | Publication date (day/month/year) | Patent family member(s) | | | Publication date (day/month/year) |
|---|---|---|---|---|---|---|---|
| JP | 2007-20931 | A | 01 February 2007 | (Family: none) | | | |
| JP | 2019-1154 | A | 10 January 2019 | US | 2018/0355144 | A1 | |
| | | | | EP | 3415563 | A1 | |
| WO | 2021/200137 | A1 | 07 October 2021 | (Family: none) | | | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006046496 A1
- US 6599989 B2
- US 6469080 B2
- EP 2163302 A1
- US 20080161512 A1
- WO 2007004529 A1
- WO 2012023433 A1
- US 4893999 A
- US 6906159 B2
- US 7091253 B2
- US 7741400 B2
- US 8519212 B2
- JP 2005036100 A
- US 6987151 B2
- WO 2011126079 A1
- WO 2006100300 A1
- WO 2011025012 A1
- WO 2011025013 A1
- WO 2011111657 A1
- US 7183456 B2
- US 20100062252 A1
- JP 2023143714 A

**Non-patent literature cited in the description**

- Non-Woven Standard Procedures. 2015